# EUROPEAN PATENT SPECIFICATION

(11) **EP 1 021 150 B1**
(45) Date of publication and mention of the grant of the patent: **18.12.2002**
(21) Application number: 98950792.6
(22) Date of filing: 01.10.1998
(51) Int. Cl.: A61F 13/15

(54) **DISPOSABLE TRAINING PANT WITH IMPROVED DISPOSAL DEVICE HAVING A ONE-PIECE DISPOSAL FEATURE**
WEGWERFERZIEHUNGSHÖSCHEN MIT EINEM EINSTÜCKIG AUSGEBILDETEN ELEMENT ZUM WEGWERFEN DES VERSCHMUTZTEN ARTIKELS
CULOTTES D'EXERCICE A DISPOSITIF JETABLE AMELIORE

(30) Priority: 06.10.1997 US 944813
(43) Date of publication of application: 26.07.2000
(73) Proprietor: THE PROCTER & GAMBLE COMPANY, Cincinnati, Ohio 45202 (US)
(72) Inventor: KING, Namy, Mariottoni, Wyoming, OH 45215 (US)
(74) Representative: Hirsch, Uwe Thomas
(86) International application number: US9820514
(87) International publication number: WO99017693

(56) References cited:
- DE-A- 19 654 456
- DE-U- 9 414 056
- US-A- 5 575 784

## Description

### FIELD OF THE INVENTION

The present invention relates to disposable garments having fixed sides, which are placed in position on the wearer by inserting the wearer's legs into the leg openings and sliding the disposable garment into position about the wearer's lower torso. Examples of such disposable garments would include disposable underwear for children (e.g., toddlers) or adults, and disposable panties which may be used with catamenial devices such as tampons or sanitary napkins. The present invention relates more particularly to disposable absorbent articles such as training pants, incontinent garments (parities or briefs), and the like, having an improved disposal device that provides for convenient disposal.

### BACKGROUND OF THE INVENTION

Infants and other incontinent individuals wear disposable absorbent articles to receive and contain urine and other bodily exudates. Absorbent articles having fixed sides, e.g. disposable training pants, have been popular for use on toilet-training children. An example of a disposable training pant is described in U.S. Pat. No. 5,246,433 issued to Hasse et al. on Sept. 21, 1993.

The use of adhesive disposal systems for securing a training pant in a configuration for disposal is well known in the art. While prior art adhesive disposal systems are fairly effective in securing a training pant in a configuration for disposal, they do have shortcomings. One shortcoming is that they do not effectively seal the training pant to contain the contents within the soiled training pant. Many prior art adhesive disposal systems do not effectively seal the leg openings to contain the contents within the soiled training pant once the training pant is in a rolled-up configuration.

As a solution to those disposal systems having one member for disposal of a garment, U.S. Patent No. 5,575,784 issued to Ames-Ooten, et al. on Nov. 19, 1996 provides a disposable garment having a disposal device that is positioned parallel to the longitudinal axis of the garment. In use, the disposal device is separated along its frangible section to form a two-piece member which is then usable for disposal and sealing of the disposable garment after its use. However, Ames-Ooten must split into two pieces to operate and is not oriented horizontally on the chassis, that is to say, it is not oriented parallel to the transverse axis.

It is therefore, an object of the present invention to provide a disposable garment with a one-piece disposal feature that will effectively contain the contents within the soiled training pant.

It is a further object herein to orient a one-piece disposal feature parallel to the transverse axis (i.e., horizontally) on the outer layer of the training pant garment so as to avoid undue stress and strain on the one-piece disposal feature caused by the folded training pant garment.

### SUMMARY OF THE INVENTION

Accordingly, the present invention provides a disposable garment having a transverse axis and a longitudinal axis. The disposable garment further comprises a chassis having a front portion, a rear portion opposed to said front portion, and a crotch portion positioned between said front portion and said rear portion. The chassis also comprises an outer layer and an inner layer. Seams join said front portion to said rear portion so as to form two leg openings and a waist opening. A disposal device joins the outer layer for allowing the disposable garment to be secured in a configuration that provides convenient disposal of the disposable garment. The disposal device comprises a one-piece disposal feature horizontally oriented and parallel to said transverse axis. The one-piece disposal feature has a pair of mutually opposed ends used for keeping the garment in a folded position at its disposal. Such opposed ends have attachment or securement means for securing the garment in its folded position prior to and during disposal. The one-piece disposal feature may be attached to said outer layer by a central portion or it may preferably be attached to the outer layer without a central portion.

In one preferred embodiment the one-piece disposal feature is positioned into a folded configuration prior to its use, e.g., into a z-fold. The one-piece disposal feature may comprise material selected from the group consisting of polyethylene, polypropylene, elastics, nonwovens and combinations thereof. Preferably, each one-piece disposal feature comprises at least one attachment device located at or near the ends of the one-piece disposal feature for securing the disposable garment in a folded configuration and another separate securement device for securing the one-piece disposal feature to the outer layer of the chassis. Suitable attachment devices herein preferably comprise one or more elements selected from the group consisting of adhesives, hooks, loops and combinations thereof.

In another preferred embodiment, the chassis of the disposable garment may additionally comprise an absorbent assembly secured to the inner layer. Further, the absorbent assembly may comprise a topsheet, a backsheet secured to the topsheet, and an absorbent core interposed between the topsheet and the backsheet, the backsheet being secured to the inner layer. The front portion of the garment has an end edge, longitudinal side edges, and leg edges. The rear portion of the garment has an end edge, longitudinal side edges, and leg edges.

### BRIEF DESCRIPTION OF THE DRAWINGS

While the specification concludes with claims particularly pointing out and distinctly claiming the subject matter which is regarded as forming the present invention, it is believed that the invention will be better understood from the following description taken in conjunction with the accompanying drawings, in which:
FIG. 1 is a perspective view of the disposable training pant embodiment of the present invention in a typical in-use configuration as it would be applied to a wearer;
FIG. 2 is a plan view of the chassis of the disposable training pant embodiment of the present invention having portions cut away to reveal the underlying structure, the surface which will form the outer surface of the disposable garment facing away from the viewer;
FIG. 3 is a plan view of the disposable training pant illustrating the disposal device;
FIG. 3A is a cross-sectional view of the embodiment in FIG. 3;
FIG. 4 is a plan view showing the disposable training pant of FIG. 3 in its disposal configuration;
FIG. 5 is an enlarged fragmented perspective illustration of the disposable training pant illustrating the disposal device; and
FIG. 6 is an enlarged fragmented perspective illustration of the disposable training pant illustrating the disposal device.

### DETAILED DESCRIPTION OF THE INVENTION

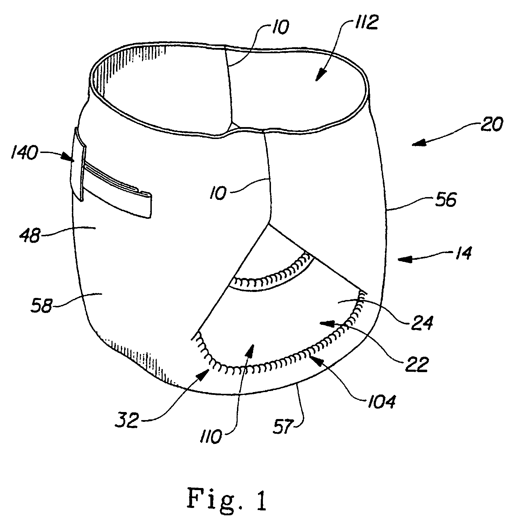

A unitary disposable garment is one which is intended to be discarded after it is used (i.e., it is not intended to be laundered or otherwise restored or reused), and which does not require separately manipulative parts such as a separate chassis and separate ear flaps. The disposable garment herein may be provided with an absorbent assembly which is placed in close proximity to the body of the wearer to absorb and contain the various exudates discharged from the body. A preferred embodiment of the unitary disposable garment of the present invention, disposable training pant 20, is shown in FIG. 1. The training pant 20 of FIG. 1, comprises a chassis 14, side seams 10, leg openings 110, a waist opening 112, an absorbent assembly 22, and a disposal device 140.

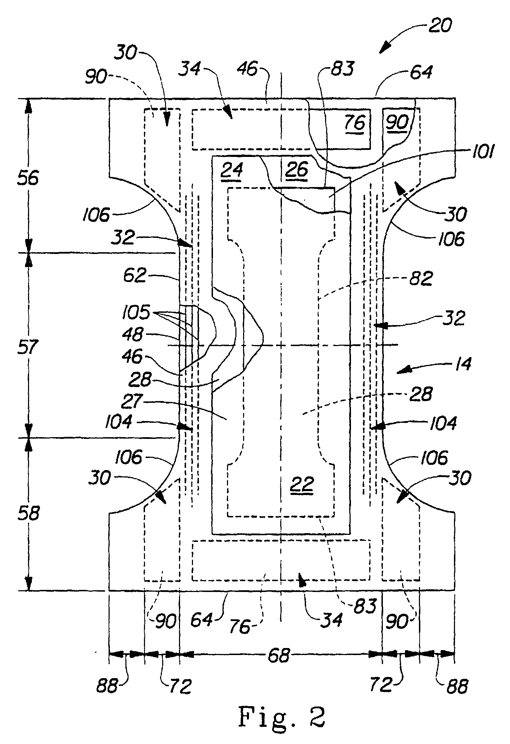

FIG. 2 is a partially cut-away perspective view of the disposable training pant 20 of FIG. 1, prior to the front portion 56 and the rear portion 58 of the chassis 14 being joined together by the seams 10 (shown in Fig. 1). The chassis 14 of the present invention preferably has a symmetric, modified hour-glass shape. The chassis 14 will have at least a front portion 56, a rear portion 58, a crotch portion 57, longitudinal side regions 88, and ear flaps 72 and will comprise an elastic ear flap member 90 operatively associated with each ear flap 72 to form a laminated ear flap. The absorbent assembly 22 is secured to the chassis 14.

As shown in FIG. 2, a preferred embodiment of the chassis 14 further comprises an outer layer 48 and an inner layer 46 with the elastic ear flap members 90, elastic waistband members 76, and elastic strands 105 preferably secured between the inner layer 46 and outer layer 48.

The outer layer 48 is that portion of the chassis 14 which will form the exterior of the disposable training pant 20, i.e., that portion facing away from the wearer. The outer layer 48 is compliant, soft feeling, and non-irritating to the wearer's skin. A suitable outer layer may be manufactured from a wide range of materials, such as plastic films; or woven or non-woven webs of natural fibers (e.g. wood or cotton fibers), synthetic fibers (e.g. polyester or polypropylene fibers), or a combination of natural and synthetic fibers. Preferably, the outer layer 48 is hydrophobic and is made of a material containing a significant amount of thermoplastic fibers, typically 50% or more, preferably 100%. Preferably the outer layer is a carded nonwoven web of polypropylene fibers. A suitable outer layer is Series 6700 Nonwovens manufactured by Scott Nonwovens of Landisville, N.J.

The inner layer 46 is that portion of the chassis 14 which will form the interior of the chassis 14, and will contact at least the waist and legs of the wearer. The inner layer is also compliant, soft feeling, and non-irritating to the wearer's skin. A suitable inner layer 46 may be manufactured from a wide range of materials, such as plastic films; or woven or non-woven webs of natural fibers (e.g. wood or cotton fibers), synthetic fibers (e.g. polyester or polypropylene fibers), or a combination of natural and synthetic fibers. Preferably the inner layer 46 is made of a material containing a significant amount of thermoplastic fibers, typically 50% or more, preferably 100%. Preferably the inner layer is also a carded nonwoven web of polypropylene fibers. More preferably, the inner layer 46 is made of the same material as the outer layer 48. A suitable inner layer is Series 6700 Nonwovens manufactured by Scott Nonwovens of Landisville, N.J.

The inner layer 46 is preferably positioned adjacent to the outer layer 48 and is preferably joined thereto by attachment means (not shown) such as those well known in the art. For example, the inner layer 46 may be secured to the outer layer 48 by a uniform continuous layer of adhesive, a patterned layer of adhesive, or an array of separate lines, spirals, or spots of adhesive. Adhesives which have been found to be satisfactory are manufactured by Findley Adhesives of Elm Grove, Wis. and marketed as Findley 2031. Alternatively, the attachment means may comprise heat bonds, pressure bonds, ultrasonic bonds, dynamic mechanical bonds, or any other suitable attachment means or combinations of these attachment means as are known in the art. As used herein, the term "joined" encompasses configurations whereby an element is directly secured to the other element by affixing the element directly to the other element, and configurations whereby the element is indirectly secured to the other element by affixing the element to intermediate member(s) which in turn are affixed to the other element. In a preferred embodiment of the present invention, the inner layer 46 and the outer layer 48 are indirectly joined together by directly joining them to the elastic ear flap members 90, elastic waistband member 76, and elastic strands 105 and are joined directly to each other in the areas extending beyond the elastic ear flap members 90, elastic waistband members 76, and elastic strands 105.

In a preferred embodiment of the present invention, at least a portion of the chassis inner and outer covers 46, 48 will be subjected to mechanical stretching in order to provide a "zero strain" stretch laminate that forms the elasticized ear flaps 30. Thus, the inner and outer layers 46, 48 are preferably elongatable, most preferably drawable, but not necessarily elastomeric, so that the inner and outer layers 46, 48 will, upon mechanical stretching, be at least to a degree permanently elongated such that they will not fully return to their original undistorted configuration. In preferred embodiments, the inner and outer layers 46, 48 can be subjected to mechanical stretching without undue rupturing or tearing. Thus, it is preferred that the inner and outer covers 46, 48 have a low cross-machine direction (lateral direction) yield strength.

The chassis 14 of the disposable training pant 20 preferably further comprises elasticized leg cuffs 32 for providing improved containment of liquids and other body exudates. Each elasticized leg cuff 32 may comprise several different embodiments for reducing the leakage of body exudates in the leg regions. (The leg cuff can be and is sometimes also referred to as leg bands, side flaps, barrier cuffs, or elastic cuffs.) U.S. Pat. No. 3,860,003 issued to Buell on Jan. 14, 1975, describes a disposable diaper which provides a contractible leg opening having a side flap and one or more elastic members to provide an elasticized leg cuff (gasketing cuff). U.S. Pat. No. 4,9089,803 issued to Aziz and Blaney on Mar. 20, 1990, describes a disposable diaper having "stand-up" elasticized flaps (barrier cuffs) to improve the containment of the leg regions. U.S. Pat. No. 4,695,278 issued to Lawson on Sept. 22, 1987, describes a disposable diaper having dual cuffs including a gasketing cuff and a barrier cuff. U.S. Pat. No. 4,704,115 issued to Buell on Nov. 3, 1987, discloses a disposable diaper or incontinent garment having side-edge-leakage-guard gutters configured to contain free liquids within the garment. While each elasticized leg cuff 32 may be configured so as to be similar to any of the leg bands, side flaps, barrier cuffs, or elastic cuffs described above, it is preferred that each elasticized leg cuff 32 comprise at least a side flap 104 and one or more elastic strands 105.

The chassis 14 of the disposable training pant 20 further preferably comprises an elasticized waistband 34 disposed adjacent the end edge 64 of the disposable training pant 20 in at least the rear portion 58, and more preferably has an elasticized waistband 34 disposed in both the front portion 56 and the rear portion 58. The waistband of the disposable training pant 20 is that portion which is intended to be placed adjacent the wearer's waist. The elasticized waistband 34 provides a member that maintains a defined area of coverage, contacts the wearer's waist, and is elastically extensible in at least the lateral direction so as to dynamically fit against the waist of the wearer and to dynamically conform to the waist of the wearer so as to provide improved fit. Thus, the waistband is generally that portion of the disposable training pant 20 extending from the end edge 64 of the disposable training pant 20 to at least the waist edge 83 of the absorbent core 28. While the elasticized waistband 34 can comprise a separate element affixed to the chassis 14 of the disposable training pant 20, the waistband is preferably an extension of other elements of the disposable training pant 20 such as the inner layer 46, the outer layer 48, or any combination of these elements and an elastomeric material joined thereto. Alternatively, the topsheet 24 and the backsheet 26 of the absorbent assembly 22, may extend beyond the edges of the absorbent core 28 and have an elastomeric material joined thereto to form an elasticized waistband. Disposable training-pants are often constructed so as to have two elasticized waistbands; one positioned in the front portion 56 and one positioned in the rear portion 58. The disposable training pant 20 at least has an elasticized waistband 34 disposed in at least the central region 68 of the rear portion 58. Preferably, as shown in FIG. 2, another elasticized waistband is disposed in the front portion 56. Preferably both elasticized waistbands 34 are disposed between the elasticized ear flaps 30.

The elasticized waistband 34 may be constructed in a number of different configurations including those described herein with regard to the elasticized side panels. In a preferred embodiment of the present invention shown in FIG. 2, the elasticized waistband 34 comprises an elastic waistband member 76 interposed between the inner cover 46 and the outer cover 48 and operatively associated with either or both the inner cover 46 and the outer cover 48 to gather the front portion 56 and rear portion 58 of the disposable training pant 20. An example of such an elasticized waistband for use herein is the elasticized waistband disclosed in U.S. Pat. No. 4,515,595 which issued to Kievit and Osterhage on May 7, 1985.

Any suitable elastomeric material as known in the art may be used as the elastic waistband member 76 of the present invention. Examples of suitable elastomeric materials include elastomeric films, elastomeric foams such as polyurethane foams or crosslinked natural rubber foams; formed elastic scrim; elastomeric films such as heat shrinkable elastic materials; elastomeric film laminates such as a laminate of a heat-shrinkable elastomeric film and a resilient member; elastomeric stretch laminates such as "zero strain" stretch laminates or mechanically stretched pretensioned stretch laminates; and elastic strands made from rubber, LYCRA®, or other materials. In a preferred embodiment, the elastic waistband member 76 comprises a heat shrinkable elastomeric film.

In an alternative embodiment, the elasticized waistbands 34 and the elasticized ear flaps 30 can be formed by securing a single piece of elastomeric material to the disposable garment 20 in both the ear flaps 72 and central region 68 of the front portion 56. Thus, the elasticized waistband 34 and the elasticized ear flaps 30 can be formed from the same piece of material to form a unitary structure.

In a preferred embodiment, the chassis 14 comprises elasticized ear flaps 30 in the front portion 56 and rear portion 58. The elasticized ear flaps 30 are unitary elements of the chassis, i.e., they are not separately manipulative elements secured to the chassis, but rather are formed from and are extensions of the chassis materials. The elasticized ear flaps 30 provide an elastically extensible feature that provides a more comfortable and contouring fit by initially conformably fitting the disposable garment to the wearer and sustaining this fit throughout the time of wear well past when the disposable garment has been loaded with exudates since the elasticized ear flaps allow the sides of the disposable garment to expand and contract.

As shown in FIG. 2, each ear flap 72 comprises that portion of the chassis 14 that extends laterally outwardly from and along the central region 68 of the chassis 14 to the longitudinal side region 88 of the chassis 14. The ear flap 72 generally extends longitudinally from the end edge 64 of the chassis 14 to the portions of the longitudinal edge 62 of the chassis 14 that forms the leg opening (this segment of the longitudinal edge 62 being designated as leg edge 106). In a preferred embodiment of the present invention, each ear flap is formed by the portions of the inner layer 46 and the outer layer 48 that extend beyond the central region 68 of the chassis 14.

In a preferred embodiment of the present invention, the elastic ear flap members 90 are operatively associated with the chassis 14 in the ear flaps 72, preferably between the inner layer 46 and the outer layer 48, so that the elastic ear flap members 90 allow the elasticized ear flaps 30 to be elastically extensible in the lateral direction (laterally elastically extensible). As used herein, the term "elastically extensible" means a segment or portion of the chassis that will elongate in at least one direction (preferably the lateral direction for the ear flaps and the waistbands) when tensional forces (typically lateral tensional forces for the ear flaps and the waistbands) are applied, and will return to about its previous size and configuration when the tensional forces are removed. Generally, elastomeric materials useful in the present invention will contractively return to at least about 75% of their original configuration within about 5 seconds or less upon stretch and immediate release thereof (i.e., a "snappy" elastic).

In an especially preferred embodiment, the elastic ear flap member 90 is operatively associated in the ear flap 72 by joining the elastic ear flap member 90 to the inner layer 46, outer layer 48, or both while the elastic ear flap member 90 is in a substantially untensioned condition. At least a portion of the resultant composite elastomeric laminate containing the elastic ear flap member 90 is then subjected to mechanical stretching sufficient to permanently elongate the inner layer and the outer layer components (nonelastic components) of the laminate. The composite elastomeric laminate is then allowed to return to its substantially untensioned condition. The elasticized ear flap is thus formed into a "zero strain" stretch laminate. (Alternatively, the elastic ear flap member could be operatively associated in a tensioned condition and then subjected to mechanical stretching; although this is not preferred as a "zero strain" stretch laminate.) As used herein, the term "zero strain" stretch laminate refers to a laminate comprised of at least two plies of material which are secured to one another along at least a portion of their coextensive surfaces while in a substantially untensioned ("zero strain") condition; one of the plies comprising a material which is stretchable and elastomeric (i.e., it will return substantially to its untensioned dimensions after an applied tensile force has been released) and a second ply which is elongatable (but not necessarily elastomeric) so that upon stretching the second ply will be, at least to a degree, permanently elongated so that upon release of the applied tensile forces, it will not fully return to its original undeformed configuration. The resulting "zero strain" stretch laminate is thereby rendered elastically extensible, at least up to the point of initial stretching, in the direction of initial stretching. Examples of such "zero strain" stretch laminates are disclosed in U.S. Pat. No. 2,075,189 issued to Galligan, et al. on Mar. 30, 1937; U.S. Pat. No. 3,025,199 issued to Harwood on Mar. 13, 1962; U.S. Pat. No. 4,107,364 issued to Sisson on Aug. 15, 1978; U.S. Pat. No. 4,209,563 issued to Sisson on Jun. 24, 1980; and U.S. Pat. No. 4,834,741 issued to Sabee on May 30, 1989.

Particularly preferred methods and apparatus used for making "zero strain" stretch laminates out of the inner layer, outer layer, and an elastomeric member positioned between the same, use meshing corrugated rolls to mechanically stretch the components. A discussion of suitable apparatus and methods for mechanically stretching portions of a diaper is contained in the hereinbefore referenced U.S. Pat. No. 4,107,364 issued to Sisson on Aug. 15, 1978 and U.S. Pat. No. 4,834,741 issued to Sabee on May 30, 1989. Particularly preferred apparatus and methods are disclosed in U.S. Pat. No. 5,167,897 issued to Gerald M. Weber et al. on Dec. 1, 1992; U.S. Pat. No. 5,156,793 issued to Kenneth B. Buell et al. on Oct. 20, 1992; and U.S. Pat. No. 5,143,679 issued to Gerald M. Weber et al. on Sept. 1, 1992.

In a preferred embodiment of the chassis as shown in FIG. 2, the longitudinal-side region 88 is that portion of the chassis 14 that extends laterally outwardly from the ear flap 72 to the longitudinal edge 62 of the chassis 14. The longitudinal side region 88 generally extends longitudinally from the end edge 64 of the chassis 14 to the portion of the longitudinal edge 62 of the chassis 14 that forms the leg opening (this segment of the longitudinal edge 62 being designated as leg edge 106). While the longitudinal side region 88 can comprise a separate element affixed to the ear flap 72 of the chassis 14, the longitudinal side region is preferably an extension of other elements of the chassis 14 such as the inner layer 46, the outer layer 48, the topsheet 24 or the backsheet 26 or any combination of these elements. In a preferred embodiment of the present invention each longitudinal side region 88 is formed by portions of the inner layer 46 and outer layer 48 that extend beyond the ear flap 72.

Referring again to FIG. 1, seams 10 are preferably formed by bonding together the longitudinal side regions 88 of the front portion 56 with the longitudinal side regions 88 of the rear portion 58. The seam 10 can be formed in a number of different ways. For example the seam 10 can be formed by bonding together portions of outwardly extending longitudinal side regions 88 to form an outwardly extending fin seam, bonding together portions of inwardly extending longitudinal side regions 88 to form an inwardly extending fin seam, the longitudinal side regions 88 may be bonded together using any other seam configurations that are well known in the art. The bonding can be by any suitable means well known in the art appropriate for the specific material employed in the longitudinal side region 88 of the chassis 14; thus sonic sealing, heat sealing, adhesive bonding, sewing, and the like may be appropriate techniques. Examples of suitable seaming techniques are disclosed in U.S. Pat. No. 4,355,425 issued to Jones, et al. on Oct. 26, 1982; U.S. Pat. No. 4,619,649 issued to Roberts on Oct. 28, 1986; U.S. Pat. No. 4,909,804 issued to Douglas, Sr. on Mar. 20, 1990; and U.S. Pat. No. 5,246,433 issued to Hasse et al. on Sept. 21, 1993.

The training pant 20 will also comprise an absorbent assembly 22. The absorbent assembly 22 of the disposable training pant 20 is an insert, i.e. an element formed separately from the chassis and inserted therein. The absorbent assembly 22 is any absorbent device which is generally compressible, conformable, non-irritating to the wearer's skin, and capable of absorbing and retaining liquids such as urine and other certain body exudates.

As shown in FIG. 2, the absorbent assembly 22 of the disposable training pant 20 preferably comprises an absorbent core 28 and an outer covering layer comprising a topsheet 24 and a backsheet 26. The absorbent assembly 22 is preferably positioned adjacent the inner layer 46 and is preferably joined thereto by suitable attachment means (not shown) such as those well known in the art (e.g., adhesive, ultrasonic bonding, etc.). Suitable attachment means herein are described more fully hereinbelow with respect to joining the backsheet 26 to the absorbent core 28.

The absorbent core 28 may be any absorbent device which is generally compressible, conformable, non-irritating to the wearer's skin, and capable of absorbing and retaining liquids such as urine and other certain body exudates. The absorbent core 28 has a garment surface 100 (not shown), a body surface 101, side edges 82 and end edges 83.

The absorbent core 28 may be manufactured in a wide variety of sizes and shapes (e.g., rectangular, hourglass, "T"-shaped, asymmetric, etc.) and from a wide variety of liquid-absorbent materials commonly used in disposable diapers and other absorbent articles such as comminuted wood pulp which is generally referred to as airfelt. Examples of other suitable absorbent materials include creped cellulose wadding, meltblown polymers including coform, cross-linked cellulosic fibers, tissue including tissue wraps, absorbent foams, absorbent sponges, superabsorbent polymers, absorbent gelling materials, or any equivalent material or combinations of materials. The configuration and construction of the absorbent core may also be varied (e.g., the absorbent core may have varying caliper zones, a hydrophilic gradient, a superabsorbent gradient, or lower average density and lower average basis weight acquisition zones; or may comprise one or more layers or structures). The total absorbent capacity of the absorbent core 28 should, however, be compatible with the design loading and the intended use of the disposable training pant 20. Further, the size and absorbent capacity of the absorbent core 28 may be varied to accommodate wearers ranging from infants through adults.

A preferred embodiment of the absorbent assembly 22 has a symmetric, modified hour-glass shape absorbent core 28. While a preferred embodiment of the absorbent assembly 22 has a modified hourglass-shaped absorbent core 28, it should be understood that the size, shape, configuration and total absorbent capacity of the absorbent core 28 may be varied to accommodate wearers ranging from infants to adults. Therefore, the dimensions, shape and configuration of the absorbent core may be varied (e.g., the absorbent core may have a varying caliper, or a hydrophilic radiant, or may or may not contain absorbent gelling materials). An exemplary absorbent structure for use as the absorbent core 28 of the present invention that has achieved wide acceptance and commercial success is described in U.S. Pat. No. 4,610,678 issued to Weisman and Goldman on Sept. 9, 1986. U.S. Pat. No. 4,673,402 issued to Weisman, Houghton and Gellert on Jun. 16, 1987; U.S. Pat. No. 4,834,735 issued to Alemany and Berg on May 30, 1989; and U.S. Pat. No. 4,888,231 issued to Angstadt on Dec. 19, 1989 also describe absorbent structures that are useful in the present invention. The absorbent core 28 is preferably a batt of airfelt and particles of absorbent gelling material, about 13 centimeters wide (lateral dimension), about 37 centimeters long (longitudinal dimension) and approximately 8 centimeters across the narrowest part of the crotch portion 57. Preferably, the portion of the absorbent core that will be generally located in the front portion 56 and crotch portion 57 will have a higher basis weight than the portion of the absorbent core that will be generally located in the rear portion 58. More preferably, the portion of the absorbent core that will be generally located in the front portion 56 and crotch portion 57 will have a basis weight three times the basis weight of the portion of the absorbent core that will be generally located in the rear portion 58. In a preferred embodiment of the absorbent core 28, about 25.4 centimeters of the absorbent core's length will be generally located in the front portion 56 and crotch portion 57 and will have a basis weight of about 0.69 grams per square inch (0.119 m/cm²) and 11.4 centimeters of the absorbent core's length will be generally located in the rear portion 58 and will have a basis weight of about 0.23 grams per square inch (0.036 g m/cm²).

The backsheet 26 is positioned adjacent the garment surface 100 of the absorbent core 28 and is preferably joined thereto by an attachment means (not shown) such as those well known in the art. For example, the backsheet 26 may be secured to the absorbent core 28 by a uniform continuous layer of adhesive, a patterned layer of adhesive, or an array of separate lines, spirals, or spots of adhesive. Adhesives which have been found to be satisfactory are manufactured by Century Adhesives, Inc. of Columbus, Ohio and marketed as Century 5227; and by H. B. Fuller Company of St. Paul, Minn. and marketed as HL-1258. The attachment means will preferably comprise an open pattern network of filaments of adhesive as is disclosed in U.S. Pat. No. 4,573,986 entitled "Disposable Waist-Containment Garment", which issued to Minetola and Tucker on Mar. 4, 1986. An exemplary attachment means of an open pattern network of filaments comprises several lines of adhesive filaments swirled into a spiral pattern such as is illustrated by the apparatus and methods shown in U.S. Pat. No. 3,911,173 issued to Sprague, Jr. on Oct. 7, 1975; U.S. Pat. No. 4,785,996 issued to Ziecker, et al. on Nov. 22, 1978; and U.S. Pat. No. 4,842,666 issued to Werenicz on Jun. 27, 1989. Alternatively, the attachment means may comprise heat bonds, pressure bonds, ultrasonic bonds, dynamic mechanical bonds, or any other suitable attachment means or combinations of these attachment means as are known in the art.

The backsheet 26 is impervious to liquids (e.g., urine) and is preferably manufactured from a thin plastic film, although other flexible liquid impervious materials may also be used. As used herein, the term "flexible" refers to materials which are compliant and will readily conform to the general shape and contours of the human body. The backsheet 26 prevents the exudates absorbed and contained in the absorbent core 28 from wetting articles which contact the disposable training pants 20 such as bedsheets and undergarments. The backsheet 26 may thus comprise a woven or nonwoven material, polymeric films such as thermoplastic films of polyethylene or polypropylene, or composite materials such as a film-coated nonwoven material. Preferably, the backsheet is a film having a thickness of from about 0.012 mm (0.5 mil) to about 0.051 mm (2.0 mils).

The size of the backsheet 26 is dictated by the size of the absorbent core 28 and the exact disposable garment design selected. In a preferred embodiment, the backsheet 26 will wrap around at least the absorbent core and possibly over the edge portions of the topsheet 24 in at least the crotch portion 57, so that the elasticized leg cuff 32 will be free from any backsheet material, and, thus are not inhibited by the backsheet material. Alternatively, the topsheet 24 may wrap around the core and under the edge portion of the backsheet 26 in at least the crotch portion 57, or the topsheet 24 and backsheet 26 may be "side-notched" in the crotch portion 57 so that the elasticized leg cuffs 32 are not inhibited by the backsheet material.

The topsheet 24 is positioned adjacent the body surface 101 of the absorbent core 28 and is preferably joined thereto and to the backsheet 26 by an attachment means (not shown) such as those well known in the art. Suitable attachment means are described with respect to joining the backsheet 26 to the absorbent core 28. In a preferred embodiment of the present invention, the topsheet 24 and the backsheet 26 are joined directly to each other in the areas extending beyond the absorbent core 28 and are indirectly joined together by directly joining them to the absorbent core 28 by the attachment means (not shown).

The topsheet 24 is compliant, soft feeling, and non-irritating to the wearer's skin. Further, the topsheet 24 is liquid pervious permitting liquids (e.g., urine) to readily penetrate through its thickness. A suitable topsheet may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. Preferably, the topsheet 24 is made of hydrophilic material comprising about 20% to 30% rayon so as to feel wet and signal a discharge of urine to a toilet training child.

There are a number of manufacturing techniques which may be used to manufacture the topsheet 24. For example, the topsheet 24 may be a nonwoven web of fibers. When the topsheet comprises a nonwoven web, the web may be spunbonded, carded, wet-laid, meltblown, hydroentangled, combinations of the above, or the like. A preferred topsheet is carded and thermally bonded by means well known to those skilled in the fabrics art. A suitable topsheet is manufactured by Fiberweb North America and available as 80/20 polypropylene/rayon carded thermally bonded nonwoven.

### The Disposal Device and One-Piece Disposal Feature

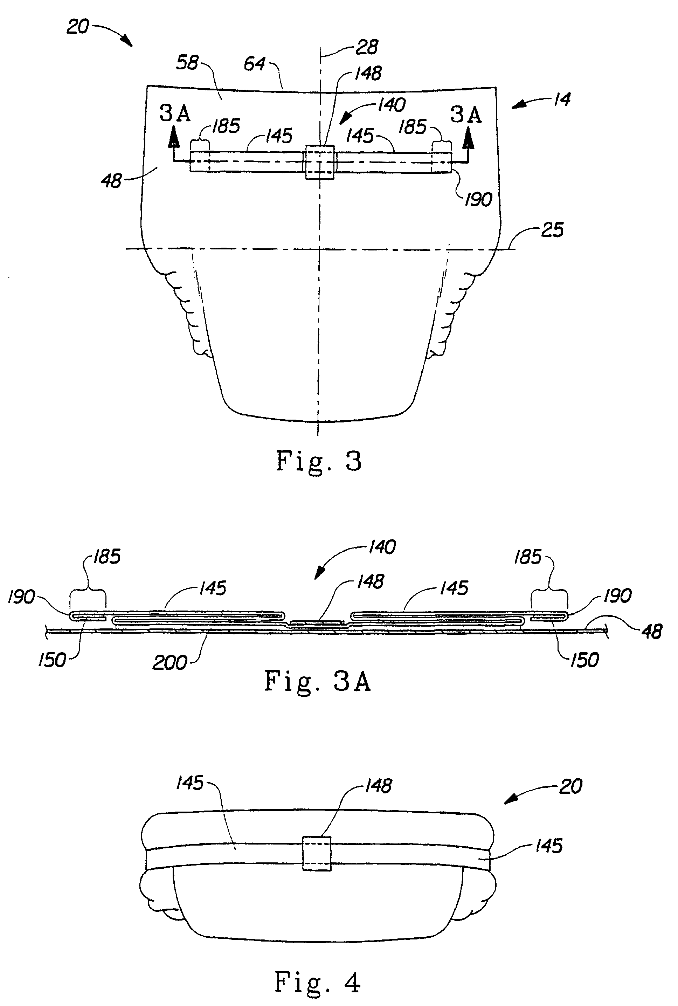

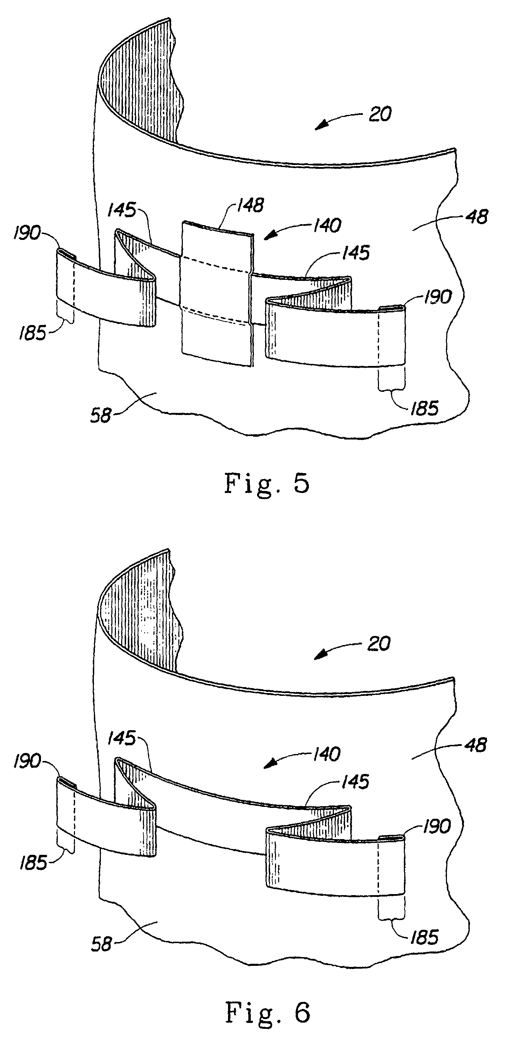

Referring now to FIGS. 1 and 3, the training pant 20 comprises a disposal device 140 having a one-piece disposal feature 145 or one-piece feature 145 or feature 145 for allowing the training pant 20 to be secured in a folded, i.e., disposal configuration so as to provide convenient disposal of the training pant 20. In addition, the desired disposal configuration herein is one in which the leg openings are substantially sealed against leakage. More specifically, the one-piece disposal feature 145 may be anchored to the outer layer 48 of the chassis 14 by a securement device 200 (shown in Figs. 3A and 6) placed at any length along the back of the one-piece disposal feature 145 (thus creating a one-piece disposal device 140) or at least a portion of the one-piece disposal feature 145 may comprise a portion of, i.e., be embedded within, the outer layer 48 of the chassis. In the first instance, the securement device 200 may be any of a number of securement devices known in the art such as adhesives, hooks, loops, ultrasonic bonding, thermal bonding, and other mechanical attachment and/or fastening device known in the art and combinations thereof. In the second instance, at least a portion of the one-piece disposal device 140 may be formed from at least a portion of the material of the chassis outer layer 48.

The disposal device 140 may be positioned anywhere on the chassis 14 so long as it secures the training pant 20 in a desired configuration herein for disposal. For example, the disposal device 140 may be positioned on the outer layer 48 in the front portion 56 or on the outer layer 48 in the rear portion 58. The disposal device 140 is shown in FIGS. 1 and 3 positioned on the outer layer 48 in the rear portion 58.

In one embodiment herein, the disposal device 140 comprises a one-piece disposal feature 145 that is anchored or secured to the outer layer 48 of the chassis 14 by a central portion 148 (as shown in Figs. 1 and 3-5). The central portion 148 may comprise any one or a combination of a number of materials compatible with the one-piece disposal feature 145 and training pant 20 herein. For example, the central portion 148 may comprise polyethylene, polypropylene, adhesive tapes, mechanical fastening elements (e.g., hooks and/or loops), nonwovens, elastomeric films and combinations thereof. The importance of providing a central portion 148 to tack down the one-piece disposal feature 145 is one, the feature 145 may be securely attached to the chassis outer surface 48 without further attachment elsewhere along the one piece disposal feature 145 and two, such attachment of the one-piece feature 145 primarily and most strongly at the central portion 148 allows the one-piece feature 145 a greater range of motion about the folded garment where necessary for efficient disposal thereof--especially where the one-piece feature 145 is secured to the outer layer 48 of the garment 20 only by the central portion 148.

The one-piece disposal feature 145 is oriented parallel to the transverse axis 25. This orientation is critical to the design of the one-piece feature 145. That is, in folding and then binding or securing the chassis 14 after use, the leg openings are best closed and secured by a binding device (i.e., the one-piece disposal feature) that is oriented and operating parallel to the transverse axis 25; i.e., oriented and operating in the cross-machine direction. While not wishing to be bound by any particular theory, it is believed that the combination of orientation and operation of the one-piece disposal feature 145 allows greater flexibility and less stress and strain on the feature 145 thereby leading to less potential failure in its operation; i.e., less opportunity for a folded and securely closed training pant 20 herein to break the bonds of the one-piece disposal feature 145 and become unfolded once the training pant 20 is disposed of in a disposal receptacle. Furthermore, it is believed that the horizontal orientation of the one-piece disposal feature 145 provides the most effective retaining capacity of the folded chassis 40 and the greatest sealing strength of the leg openings 110 of the chassis 40. By the term "retaining capacity" it is meant herein the ability of the folded and sealed chassis to remain in such configuration without being manually unsealed and unfolded. By the term "sealing strength" it is meant herein the amount of sealing of the leg openings necessary to prevent exudate leakage from the leg openings when the chassis is in a folded and sealed state.

Figure 3A provides a cross-sectional view of the disposal device 140 whereby the one-piece disposal feature is in a folded, and in particular, a z-folded configuration. This is a preferred embodiment that allows multiple lengths of the one-piece disposal feature 145 to be designed into the disposal device 140. When used with the one-piece disposal feature 145 herein the central portion 148 is positioned centrally between the one-piece disposal feature 145. In addition, fastening members 150 incorporated within grip tabs 190 are preferably assigned onto the one-piece disposal feature 145 at its ends as is clearly shown in Fig. 3A. These fastening or attachment members 150 may consist of adhesives, hooks, loops, combinations of these and any attachment device known in the art for securing one portion of an absorbent article to another portion thereon. The fastening members 150 for the one-piece disposal feature 145 may comprise materials consisting of polyethylene, polypropylene, elastics, nonwovens and combinations of any of these materials. Preferably, each fastening member 150 comprises an elastic-nonwoven mix whereby two or more strands of elastic lie between two or more layers of nonwoven, such mix being formed like any of the elasticized waistband constructions known in the art and mentioned previously herein; e.g. U.S. Patent No. 4,515,595 issued to Kievit, et al. on May 7, 1985. In an elastic disposal feature, the ends of the feature may rotate around the central portion, allowing more flexibility when securing the diaper for disposal.

FIG. 5 shows an enlarged fragmented perspective illustration of the disposable training pant illustrating an embodiment of the disposal device 140. In particular, the one-piece disposal feature 145 is shown being pulled out of its folded configuration. Additionally, a connective region 185 herein is shown on each end of the one-piece disposal feature 145 to be folded onto a portion of the underside of the one-piece disposal feature 145 thereby forming grip tabs 190 at each end of the feature 145. In practice, when the training pant 20 is folded-up and ready for securement, the one-piece disposal feature 145 will be unfolded and/or pulled out of its pre-use position. By the term "pre-use position" it is meant herein the starting position of the one-piece disposal feature 145 before its use to seal the training pant 20 prior to its disposal. Next, the folded-over portions of the grip tabs 190 will be opened and folded back to reveal the attachment members 150 of the connective regions 185. As mentioned previously, such attachment members 150 may be any of several types of adhesives and/or mechanical devices known in the art (e.g., tape tabs and/or hooks).

While the one-piece disposal feature 145 may be positioned anywhere on the chassis 14, the one-piece disposal feature 145 is preferably disposed in the rear portion 58 away from the end edge 64 of the training pant 20. Thus, when the training pant 20 is folded or rolled up after soiling, the user secures the one-piece disposal feature 145 to the outer layer 48 or another portion of the training pant 20 to provide secure closure of the rolled-up training pant. The one-piece disposal feature 145 is preferably long enough at both ends such that the leg openings 110 may be substantially sealed against leakage, as shown in FIG. 4. Sealing the leg openings 110 substantially contains the contents within the soiled training pant 20 and preferably blocks most, if not all, of exudate leakage from the leg openings 110. The one-piece disposal feature 145 may be comprised of stretchable or extensible material, such as natural or synthetic elastics, or composite elastic materials, to allow the one-piece disposal feature 145 to extend and stretch as necessary about a folded-up training pant 20 to effect closed securement of the pant 20.

In an alternative embodiment, the one-piece disposal feature 145 is extensible, preferably in an elastic manner without the use of elastics. Specifically, the one-piece disposal feature 145 may comprise an elastomeric film or a structural elastic-like film web as disclosed in U.S. Patent No. 5,518,801, published Feb. 9, 1995, in the name of Chappell et al.

While particular embodiments of the present invention have been illustrated and described, it would be obvious to those skilled in the art that various other changes and modifications can be made without departing from the scope of the invention as defined by the appended claims.

## Claims

1. A disposable garment 20 having a transverse axis (25) and a longitudinal axis (28), comprising:
(a) a chassis (14) having a front portion (56), a rear portion (58) opposed to said front portion (56), and a crotch portion (57) positioned between said front portion (56), and said rear portion (58), said chassis (14) comprising an outer layer (48) and an inner layer (46);
(b) seams (10) joining said front portion (56) to said rear portion (58) so as to form two leg openings (110) and a waist opening (112);
(c) a disposal device (140) joined to said outer layer (48) for allowing the disposable garment to be secured in a configuration that provides disposal of the disposable garment, said disposal device (140) comprising a one-piece disposal feature (145), said one-piece disposal features (145) having a pair of mutually opposed ends not attached to said outer layer (48) of said garment so that said mutually opposed ends may freely wrap around said garment (20) and securely hold said garment (20) in a folded position when said garment is being folded for disposal, said one-piece disposal feature (145) being joined with said outer layer (48),
**characterized in that** said disposal device (140) is horizontally oriented and parallel to said transverse axis prior to use.

2. The disposable garment of Claim 1 wherein at least a portion of said one piece disposal feature (145) is attached to said outer layer (48) by a central portion (148), a securement device (200) or wherein at least a portion of said one piece disposal feature (145) is embedded within said outer layer (48).

3. The disposable garment of any of the preceding claims wherein said one-piece disposal feature (145) comprises a portion of said outer layer (48) of said chassis (14).

4. The disposable garment of any of the preceding claims wherein said one-piece disposal feature (145) is positioned into a folded configuration prior to use.

5. The disposable garment of any of the preceding claims wherein said one-piece disposal feature (145) comprises material selected from the group consisting of polyethylene, polypropylene, elastics, nonwoyens and combinations thereof.

6. The disposable garment of any of the preceding claims wherein each said mutually opposed end of said one-piece disposal feature (145) comprises a connective region (185) having grip tabs (190) thereon in a folded-over configuration.

7. The disposable garment of claim 6 wherein each said grip tab (190) comprises at least one attachment member (150) selected from the group consisting of adhesives, hooks, loops and cornbinarions thereof.

8. The disposable garment of any of the preceding claims wherein said chassis (14) additionally comprises an absorbent assembly (22) secured to said inner layer (46).

9. The disposable garment of claim 9 wherein said absorbent assembly (22) comprises a topsheet (24), a backsheet (26) secured to said topsheet (24), and an absorbent core (28) interposed between said topsheet (24) and said backsheet (26), said backsheet (26) being secured to said inner layer (46).

## Patentansprüche

1. Einwegwäsche (20) mit einer Querachse (25) und einer Längsachse (28), mit:
(a) einem Grundkörper (14) mit einem vorderen Bereich (56), einem hinteren Bereich (58), der dem vorderen Bereich (56) entgegengesetzt liegt, und einem Schrittbereich (57), der zwischen dem vorderen Bereich (56) und dem hinteren Bereich (58) positioniert ist, wobei der Grundkörper eine Außenschicht (48) und eine Innenschicht (46) aufweist;
(b) Nähten (10), die den vorderen Bereich (56) mit dem hinteren Bereich (58) verbinden, um so zwei Beinöffhungen (110) und eine Taillenöffhung (112) zu bilden;
(c) einem Wegwerfmittel (140), daß mit der Außenschicht (148) verbunden ist, damit die Einwegwäsche in einer Konfiguration festgemacht werden kann, die den Wegwurf der Einwegwäsche ermöglicht, wobei das Wegwerfmittel (140) ein einteiliges Wegwurfmerkmal (145) aufweist, wobei das einteilige Wegwurfmerkmal (145) ein Paar einander entgegengesetzte Enden aufweist, die an der Außenschicht (48) der Wäsche nicht angebracht sind, so daß sich die einander entgegengesetzten Enden frei um die Wäsche (20) herum legen können und die Wäsche (20) in einer gefalteten Position festhalten, wenn die Wäsche zum Wegwurf gefaltet ist, wobei das einteilige Wegwurfmerkmal (145) mit der Außenschicht (48) verbunden ist,
**dadurch gekennzeichnet, daß** das Wegwerfmittel (140) vor der Verwendung horizontal und parallel zur Querachse ausgerichtet ist.

2. Einwegwäsche nach Anspruch 1, in welcher wenigstens ein Bereich des einteiligen Wegwurfmerkmals (145) an der Außenschicht (48) durch einen zentralen Bereich (148). eine Befestigungseinrichtung (200) angebracht ist oder in welcher wenigstens ein Bereich des einteiligen Wegwurfmerkmals (145) in der Außenschicht (48) eingebettet ist.

3. Einwegwäsche nach einem der vorstehenden Ansprüche, in welcher das einteilige Wegwurfmerkmal (145) einen Bereich der Außenschicht (48) des Grundkörpers (14) umfaßt.

4. Einwegwäsche nach einem der vorstehenden Ansprüche, in welcher das einteilige Werwurfmerkmal (145) vor der Verwendung in einer gefalteten Konfiguration positioniert ist.

5. Einwegwäsche nach einem der vorstehenden Ansprüche, in welcher das einteilige Wegwurfmerkmal (145) Material aufweist, ausgewählt aus der Gruppe bestehend aus Polyethylen, Polypropylen, Elastics, Vliesstoffe und Kombinationen davon.

6. Einwegwäsche nach einem der vorstehenden Ansprüche, in welcher jedes einander entgegengesetzte Ende des einteiligen Wegwurfmerkmals (145) eine Verbindungsregion (185) mit Greifzungen (190) darauf in einer übereinander gefalteten Konfiguration aufweist.

7. Einwegwäsche nach Anspruch 6, in welcher jede Greifzunge (190) wenigstens ein Anbringungselement (150) aufweist, ausgewählt aus der Gruppe bestehend aus Haftmitteln, Haken, Schlingen und Kombinationen davon.

8. Einwegwäsche nach einem der vorstehenden Ansprüche, in welcher der Grundkörper (14) zusätzlich eine absorbierende Einheit (22) aufweist, die an der Innenschicht (46) festgelegt ist.

9. Einwegwäsche nach Anspruch 9, in welcher die absorbierende Einheit (22) eine Oberschicht (24), eine an der Oberschicht (24) festgelegte Unterschicht (26) und einen zwischen der Oberschicht (24) und der Unterschicht (26) angeordneten. absorbierenden Kern (28) aufweist, wobei die Unterschicht (26) an der Innenschicht (46) festgelegt ist.

## Revendications

1. Vêtement jetable (20) présentant un axe transversal (25) et un axe longitudinal (28) comprenant :
(a) un châssis (14) comportant une partie avant (56), une partie arrière (58) opposée à ladite partie avant (56) et une partie d'entrejambe (57) placée entre ladite partie avant (56) et ladite partie arrière (58), ledit châssis (14) comprenant une couche extérieure (48) et une couche intérieure (46) ;
(b) des coutures (10) réunissant ladite partie avant (56) à ladite partie arrière (58) afin de former deux ouvertures de jambe (110) et une ouverture de ceinture (112) ;
(c) un dispositif de mise au rebut (140) réuni à ladite couche extérieure (48), destiné à permettre que le vêtement jetable soit assujetti selon une configuration qui assure une mise au rebut du vêtement jetable, ledit dispositif de mise au rebut (140) comprenant un élément de mise au rebut (145) en une pièce, ledit élément de mise au rebut (145) en une pièce présentant une paire d'extrémités opposées l'une à l'autre non fixées à ladite couche extérieure (48) dudit vêtement, de sorte que lesdites extrémités opposées l'une à l'autre puissent s'enrouler librement autour dudit vêtement (20) et maintenir de manière sûre ledit vêtement (20) dans une position de pliage lorsque ledit vêtement est plié en vue d'une mise au rebut, ledit élément de mise au rebut (145) en une pièce étant réuni à ladite couche extérieure (48),
**caractérisé en ce que** ledit dispositif de mise au rebut (140) est orienté horizontalement et est parallèle audit axe transversal avant utilisation.

2. Vêtement jetable selon la revendication 1, dans lequel au moins une partie dudit élément de mise au rebut (145) en une pièce est fixée à ladite couche extérieure (48) par une partie centrale (148) ou un dispositif d'assujettissement (200), ou dans lequel au moins une partie dudit élément de mise au rebut (145) en une pièce est incorporée à l'intérieur de ladite couche extérieure (48).

3. Vêtement jetable selon l'une quelconque des revendications précédentes, dans lequel ledit élément de mise au rebut (145) en une pièce comprend une partie de ladite couche extérieure (48) dudit châssis (14).

4. Vêtement jetable selon l'une quelconque des revendications précédentes, dans lequel ledit élément de mise au rebut (145) en une pièce est placé dans une configuration de pliage avant utilisation.

5. Vêtement jetable selon l'une quelconque des revendications précédentes, dans lequel ledit élément de mise au rebut (145) en une pièce comprend un matériau choisi dans le groupe constitué par le polyéthylène, le polypropylène, les élastiques, les non-tissés et leurs combinaisons.

6. Vêtement jetable selon l'une quelconque des revendications précédentes, dans lequel chacune desdites extrémités opposées l'une à l'autre dudit élément de mise au rebut (145) en une pièce comprend une région de liaison (185) sur laquelle se trouvent des pattes de préhension (190) dans une configuration de rabattement.

7. Vêtement jetable selon la revendication 6, dans lequel chacune desdites pattes de préhension (190) comprend au moins un élément de fixation (150) choisi dans le groupe constitué par les adhésifs, les crochets, les boucles et leurs combinaisons.

8. Vêtement jetable selon l'une quelconque des revendications précédentes, dans lequel ledit châssis (14) comprend, en outre, un ensemble absorbant (22) assujetti à ladite couche intérieure (46).

9. Vêtement jetable selon la revendication 9, dans lequel ledit ensemble absorbant (22) comprend une feuille de dessus (24), une feuille de fond (26) fixée sur ladite feuille de dessus (24) et une âme absorbante (28) intercalée entre ladite feuille de dessus (24) et ladite feuille de fond (26), ladite feuille de fond (26) étant assujettie à ladite couche intérieure (46).
